# EUROPÄISCHE PATENTSCHRIFT

(11) **EP 3 535 572 B1**
(45) Veröffentlichungstag und Bekanntmachung des Hinweises auf die Patenterteilung: **06.07.2022**
(21) Anmeldenummer: 17800429.7
(22) Anmeldetag: 02.11.2017
(51) Int. Cl.: G01N 27/417, G01N 27/407, F24C 7/08

(54) **VERFAHREN ZUM STARTEN EINES KALIBRIERVORGANGS EINES SAUERSTOFFSENSORS EINES HAUSHALTSGERÄTS**
METHOD FOR STARTING A CALIBRATION PROCEDURE OF AN OXYGEN SENSOR OF A DOMESTIC APPLIANCE
PROCÉDÉ DE DÉMARRAGE D'UNE OPÉRATION D'ÉTALONNAGE D'UN CAPTEUR D'OXYGÈNE D'UN APPAREIL ÉLECTROMÉNAGER

(30) Priorität: 02.11.2016 DE 102016221446
(43) Veröffentlichungstag der Anmeldung: 11.09.2019
(73) Patentinhaber: BSH Hausgeräte GmbH, 81739 München (DE)
(72) Erfinder: LEGO, Dieter, 76137 Karlsruhe (DE); BAUER, Hans-Jürgen, 83278 Traunstein (DE)
(86) Internationale Anmeldenummer: PCT/EP2017/077995
(87) Internationale Veröffentlichungsnummer: WO 2018/083139

(56) Entgegenhaltungen:
- DE-A1-102012 201 471
- DE-B3-102010 054 607
- JP-B2- 2 851 182
- US-A- 5 457 963

## Beschreibung

Die Erfindung betrifft ein Verfahren zum Starten eines Kalibriervorgangs eines Sauerstoffsensors eines Haushaltsgeräts. Die Erfindung ist insbesondere vorteilhaft anwendbar Dampfgargeräte, z.B. auf Backöfen mit einer Dampfgarfunktion oder auf eigenständige Dampfgarer.

DE 10 2010 054 607 B3 betrifft ein Verfahren zum Betreiben eines Gargerätes mit folgenden Schritten: Ermittlung des Sauerstoffpartialdrucks über eine Lambdasonde, Bestimmung des tatsächlichen Umgebungsdrucks auf Basis des ermittelten Sauerstoffpartialdrucks und Kalibrieren des Gargerätes auf Basis des tatsächlichen Umgebungsdrucks. Unter einem "Kalibrieren" wird hier ein Erkennen des tatsächlichen Umgebungsdrucks des Gargeräts und Anpassen von Betriebsabläufen an den tatsächlichen Umgebungsdruck verstanden. Dabei wird vorausgesetzt, dass der mittels der Lambdasonde bestimmte Sauerstoffpartialdruck korrekt mit dem Luftdruck korreliert werden kann. Um einen Einfluss einer Luftfeuchtigkeit auf die Messung des Sauerstoffpartialdrucks zu verringern, kann die "Kalibrierung" zu vorgegebenen Tageszeiten durchgeführt werden. Nicht offenbart wird ein Kalibrieren der Lambdasonde, um ein Driften der Lambdasonde zu berücksichtigen.

DE 10 2012 021 928 A1 betrifft ein Verfahren und eine Vorrichtung zur Kalibrierung eines Abgassensors, der in einem Messraum angeordnet ist, wobei der Messraum in einem oder angrenzend an einem Abgaskanal einer Brennkraftmaschine vorgesehen ist. Zu Beginn einer Kalibrierphase wird im Messraum befindliches Abgas durch eine Befüllung des Messraums mit Kalibriergas verdrängt und bei Beendigung der Kalibrierphase wird Abgas in den Messraum eingeleitet bzw. diffundiert dieses dort ein.

EP 2 161 569 A2 betrifft ein Verfahren zum Kalibrieren eines von einem in einer Abgasleitung einer Brennkraftmaschine angeordneten Sensors, insbesondere eines NOx-Sensors und/oder eines Lambda-Sensors, bereitgestellten Messsignals, wobei das von einer Messspitze des Sensors ermittelte Messsignal in Abhängigkeit der NOx-Konzentration in einer Kennlinie darstellbar ist, und wobei die Kennlinie eine kontinuierlich oder diskontinuierlich abzugleichende Nullpunktabweichung aufweist. Es wird ein Verfahren zum Kalibrieren eines Sensors angegeben, das unabhängig von einem Schiebebetrieb der Brennkraftmaschine durchführbar ist. Dies wird dadurch erreicht, dass die Abgleichung im normalen Brennkraftmaschinenbetrieb durch ein Vorbeiströmen von Spülluft an der Messspitze des Sensors erfolgt. Dazu wird die Messspitze entweder aus der Abgasleitung ausgeblendet oder aber Spülluft gezielt an die Messspitze geblasen.

Es ist die Aufgabe der vorliegenden Erfindung, die Nachteile des Standes der Technik zumindest teilweise zu überwinden und insbesondere eine Möglichkeit bereitzustellen, um ein Driften eines Sauerstoffsensors eines Haushaltsgeräts zuverlässig zu erkennen und auszugleichen.

Diese Aufgabe wird gemäß den Merkmalen der unabhängigen Ansprüche gelöst. Bevorzugte Ausführungsformen sind insbesondere den abhängigen Ansprüchen entnehmbar.

Die Aufgabe wird gelöst durch ein Verfahren zum Starten eines Kalibriervorgangs eines Sauerstoffsensors eines Haushaltsgeräts mit einem Behandlungsraum und einer den Behandlungsraum verschließenden Tür, bei dem der Kalibriervorgang dann automatisch gestartet wird, wenn eine Türöffnungszeit einen zugehörigen Schwellwert erreicht oder überschritten hat.

Bei diesem Verfahren wird die Erkenntnis ausgenutzt, dass im Laufe der Lebensdauer des Sauerstoffsensors dessen Messwerte driften, so dass sich die absolute Genauigkeit der Messwerte kontinuierlich verschlechtert. Dieses typischerweise nach Wochen oder Monaten merkliche Driften unterscheidet sich sowohl von einem Sauerstoffsensor zum anderen als auch abhängig von einer Beaufschlagung mit schädlichen Substanzen wie z. B. Silikon.

Dieses Verfahren weist den Vorteil auf, dass die Drift zuverlässig erkannt und ggf. ausgeglichen werden kann. Dadurch wiederum lässt sich eine höhere absolute Messgenauigkeit des Sauerstoffsensors erreichen. Da ein Sauerstoffgehalt in einem Behandlungsraum des Haushaltsgeräts umgekehrt proportional zu einer Feuchte in dem Behandlungsraum ist, kann folglich der durch den Sauerstoffsensor gemessene Sauerstoffgehalt als ein Maß für den Feuchtegehalt in dem Behandlungsraum verwendet werden.

Dies ist insbesondere vorteilhaft, wenn der Sauerstoffsensor für eine Feuchteregelung des Garraums eingesetzt wird. Denn bei einer Verwendung des Feuchtegehalts als Führungsgröße (Sollwert) für die Feuchteregelung sind die absoluten Messwerte von Bedeutung, und das Driften der Messwerte hat einen direkten Einfluss auf die Regelgenauigkeit. Ein Kalibrieren gemäß dem beschriebenen Verfahren erhöht die Messgenauigkeit und damit die Regelgenauigkeit wieder.

Als ein weiterer Vorteil ist das Kalibrieren mit einfachen geräteeigenen Mitteln durchführbar. Es sind keine zusätzlichen Komponenten oder Prüfgase notwendig. Auch wird keine Information über externe Umgebungsparameter wie eine geodätische Höhe des Aufstellorts oder eine Uhrzeit an dem Aufstellort benötigt.

Der Sauerstoffsensor kann in den Garraum hineinragen oder mit dem Garraum gastechnisch verbunden sein.

Das Kalibrieren der Messwerte umfasst ein Berücksichtigen und ggf. Korrigieren einer Drift der Messwerte des Sauerstoffsensors. Die Drift umfasst insbesondere eine zeitliche Änderung der Messwerte bei konstanter Messumgebung. So können die Messwerte für den gleichen Sauerstoffpartialdruck oder Sauerstoffgehalt mit der Zeit wandern. Durch das Kalibrieren kann eine definierte Beziehung zwischen dem Sauerstoffpartialdruck und den Messwerten wiederhergestellt werden. Beispielsweise kann ein Messwert einem vorbekannten Sauerstoffpartialdruck oder Sauerstoffgehalt zugeordnet werden, wodurch dieser Messwert als eine neue Referenz dienen kann.

Unter einem Zustandsparameter des Haushaltsgeräts wird insbesondere ein Zustandsparameter verstanden, der dem Haushaltsgerät als solchem zugeordnet ist und nicht nur ein Umgebungsparameter wie eine Uhrzeit oder ein Aufstellort des Haushaltsgeräts ist. Durch die Abhängigkeit des Starts des Kalibriervorgangs von dem mindestens einen Zustandsparameter wird vorteilhafterweise plausibilisiert, dass eine für den Kalibriervorgang geeignete Messumgebung vorliegt. Die geeignete Messumgebung ist insbesondere eine Messumgebung mit einem ausreichend bekannten Sauerstoffgehalt. Die Zustandsparameter bzw. deren Schwellwerte knüpfen also den Kalibrierungsvorgang an Bedingungen, die einen bekannten Sauerstoffwert mit ausreichender Genauigkeit sicherstellen. Als bekannter Sauerstoffgehalt wird insbesondere der Sauerstoffgehalt atmosphärischer Luft mit einem Sauerstoffanteil von 20,95 % verwendet.

Dass ein Zustandsparameter einen vorgegebenen Schwellwert zumindest erreicht hat, kann das genaue Erreichen des vorgegebenen Schwellwerts umfassen, alternativ oder zusätzlich ein Überschreiten oder ein Unterschreiten des vorgegebenen Schwellwerts. Beispielsweise kann ein Zustandsparameter einen vorgegebenen Schwellwert zumindest erreicht haben, wenn der Wert des Zustandsparameters gleich ("="), größer gleich (">=") oder größer (">") ist als der vorgegebene Schwellwert.

Es ist eine Weiterbildung, dass der Sauerstoffsensor auch dazu verwendet wird, um Garabläufe beruhend auf einer Änderung des Feuchtegehalts während des Garablaufs zu steuern. Beispielsweise kann so ein Feuchtemaximum in dem Garraum erkannt und zur Feststellung eines Fertigstellungsgrads von Gargut verwendet werden. Bei einer solchen Verwendung des Sauerstoffsensors stellt die Drift der Messwerte kein praktisches Problem dar, da nur auf Messwertveränderungen reagiert wird und die absoluten Messwerte nicht ausgewertet werden.

Der Sauerstoffsensor kann z.B. vor oder mit Inbetriebnahme erstmals kalibriert werden (Erstkalibrierung). Das beschriebene Verfahren kann dann als eine Nachkalibrierung angesehen werden.

Es ist eine Weiterbildung, dass das Verfahren automatisch durch das Haushaltsgerät durchgeführt wird, also ohne eine Zwischenschaltung eines Nutzers. Dies ermöglicht eine besonders hohe Nutzerfreundlichkeit, da keine spezielle Handlung eines Nutzers für den Kalibriervorgang notwendig ist. Der Kalibriervorgang kann vielmehr unbemerkt im Hintergrund ablaufen. Das Verfahren kann insbesondere nur automatisch durchgeführt werden. Es ist alternativ oder zusätzlich möglich, dass das Haushaltsgerät den Nutzer auffordert, einen Kalibriervorgang durchzuführen und dazu beispielsweise die Garraumtür für eine angezeigte Zeitdauer bei kühlem Garraum zu öffnen. Eine solche Zwischenschaltung eines Nutzers kann beispielsweise dann vorgesehen sein, wenn es dem Haushaltsgerät nicht möglich war, innerhalb einer vorgegebenen Zeitdauer (z.B. einer vorgegebenen Betriebszeitdauer seit einer letzten Kalibrierung, z.B. von 100 Stunden) einen Kalibriervorgang erfolgreich durchzuführen, weil die Zustandsbedingungen nicht erfüllt waren.

Es ist eine Weiterbildung, dass das Haushaltsgerät ein Küchengerät ist, insbesondere ein Gargerät.

Es ist eine Ausgestaltung, dass das Haushaltsgerät ein Dampfgargerät ist. Das Verfahren ist hierzu besonders vorteilhaft einsetzbar, weil eine genaue Feuchteregelung ein Garergebnis erheblich verbessern kann. Das Dampfgargerät weist als Behandlungsraum einen mit Dampf beaufschlagbaren Garraum auf. Das Dampfgargerät kann ein eigenständiges Dampfgargerät sein. Es kann alternativ ein Gargerät mit einer Dampfbehandlungsfunktion sein, beispielsweise ein Backofen mit einer zusätzlichen Dampfgarfunktion ("added steam"). Der Garraum kann zur Dampfbeaufschlagung einen Dampferzeuger aufweisen, beispielsweise einen außerhalb des Garraums angeordneten Dampferzeuger (Boiler, Durchlauferhitzer usw.) oder eine in dem Garraum befindliche, heizbare Wasserschale.

Das Haushaltsgerät kann aber auch eine Spülmaschine mit einem Spülraum als dem Behandlungsraum, ein Wäschetrockner mit einem Wäschetrommelgehäuse als dem Behandlungsraum usw. sein.

Es ist eine Ausgestaltung, dass als der Sauerstoffsensor eine Lambdasonde verwendet wird. Eine Lambdasonde (auch als Lambdasensor bezeichnet) weist den Vorteil auf, dass er besonders robust, insbesondere auch gegenüber hohen Temperaturen, und zuverlässig ist.

Es ist noch eine Ausgestaltung, dass der Kalibriervorgang dann automatisch gestartet wird, wenn mehrere Zustandsparameter des Haushaltsgeräts einen jeweils vorgegebenen Schwellwert erreicht haben. Dies erhöht eine Zuverlässigkeit des Kalibriervorgangs, da nun über mehrfache, inhaltlich unterschiedliche Abfragen plausibilisiert werden kann, dass eine für den Kalibriervorgang geeignete Messumgebung vorliegt. Insbesondere haben die mehreren Zustandsparameter ihren jeweils vorgegebenen Schwellwert gleichzeitig oder innerhalb eines vorgegebenen Zeitintervalls erreicht. Das vorgegebene Zeitintervall kann beispielsweise diejenige Zeitdauer sein, die benötigt wird, um die mehreren Zustandsparameter abzufragen.

Es ist eine weitere Ausgestaltung, dass der mindestens eine Zustandsparameter des Haushaltsgeräts eine seit dem letzten Kalibriervorgang vergangene Zeitdauer umfasst und der Kalibriervorgang dann automatisch gestartet wird, wenn diese Zeitdauer einen zugehörigen Schwellwert erreicht oder überschritten hat. Diese Ausgestaltung ergibt den Vorteil, dass das Verfahren nicht häufiger als notwendig durchgeführt wird.

Es ist eine Weiterbildung, dass die Zeitdauer eine Betriebsdauer ist, was besonders lange Abstände zwischen den Kalibriervorgängen erlaubt. Die Betriebsdauer kann mittels eines Betriebsstundenzählers vorgenommen werden, der vorteilhafterweise bereits in vielen Haushaltsgeräten vorhanden ist. Der als Mindestbetriebsdauer dienende Schwellwert kann mehrere 10 Stunden betragen, z.B. 30 Stunden.

In der vorliegenden Erfindung wird der Kalibriervorgang dann automatisch gestartet, wenn die Türöffnungszeit einen zugehörigen Schwellwert erreicht oder überschritten hat. Dies ergibt den Vorteil, dass der Sauerstoffsensor sich während des Kalibrierungsvorgangs in frischer Umgebungsluft befindet, da die Umgebungsluft durch die Beschickungsöffnung in den Garraum eindringen kann und dort vorhandene Luft, ggf. andere Gase und Feuchtigkeit verdrängen kann. Die Türöffnungszeit entspricht derjenigen Zeit, die eine Garraumtür durchgehend geöffnet ist. Ein geöffneter Zustand der Garraumtür kann z.B. mittels eines Türöffnungsschalters festgestellt werden. Der Schwellwert der Türöffnungszeit entspricht insbesondere einer Mindestzeit, welche ein Garraumtür geöffnet sein sollte, um einen ausreichend gründlichen Luftaustausch zu erreichen. Die Türöffnungszeit kann z.B. 10, 20, 30 oder 60 Sekunden betragen.

Es ist eine Weiterbildung, dass die Türöffnungszeit bei geschlossener Garraumtür Null beträgt, so dass ein positives Abfragen der Türöffnungszeit umfasst, dass zum Starten des Kalibriervorgangs die Tür zum Garraum offen ist.

Es ist ferner eine Ausgestaltung, dass der mindestens eine Zustandsparameter des Dampfgargeräts eine Temperatur in dem Behandlungsraum (z.B. eine Garraumtemperatur) umfasst und der Kalibriervorgang dann automatisch gestartet wird, wenn diese Temperatur einen zugehörigen Schwellwert (Grenztemperatur) erreicht oder unterschritten hat. Dadurch wird vorteilhafterweise erreicht, dass typischerweise der Luftaustausch mit der Backofenumgebung weitestgehend abgeschlossen ist und somit in der Regel auch keine Feuchtigkeit mehr von einem Gargut abgegeben wird. Die Grenztemperatur kann z.B. zwischen 45 °C und 60 °C betragen, insbesondere ca. 50 °C.

Es ist eine Weiterbildung, dass der Kalibriervorgang dann automatisch gestartet wird, wenn die Zeitdauer seit dem letzten Kalibriervorgang ihren Schwellwert erreicht oder überschritten hat, die Türöffnungszeit ihren Schwellwert erreicht oder überschritten hat und die Garraumtemperatur ihren Schwellwert erreicht oder unterschritten hat.

Es ist auch eine Ausgestaltung, dass während des Kalibriervorgangs ein Pumpstrom einer als Sauerstoffsensor dienenden Lambdasonde gemessen wird und dann, wenn der Pumpstrom für eine vorgegebene Zeitdauer innerhalb eines vorgegebenen Wertebereichs oder Wertebands ("Schwankungsbands") verbleibt, ein Wert des Pumpstroms aus diesem Schwankungsband als ein Referenzwert des Pumpstroms ausgewählt wird. Dadurch kann auf eine einfache und zuverlässige Weise ein neuer Referenzwert des Pumpstroms bestimmt und für den Betrieb des Gargeräts, insbesondere für eine Feuchtemessung, verwendet werden. Dieser neue Referenzwert ist auf die an dem Sauerstoffsensor während des Kalibriervorgangs vorhandene Luft angepasst. Diese Ausgestaltung ist auf andere Arten von Sauerstoffsensoren mit entsprechenden Messwerten analog anwendbar.

Die vorgegebene Zeitdauer dient dazu, dass Messwerte auch zeitlich zuverlässig innerhalb des Schwankungsbands liegen. Die vorgegebene Zeitdauer kann beispielsweise mindestens 30 Sekunden betragen.

Es ist außerdem eine Ausgestaltung, dass ein mittlerer Wert des Schwankungsbands als der kalibrierte Referenzwert des Pumpstroms ausgewählt wird. Der mittlere Wert des Schwankungsbands kann insbesondere als ein Mittelwert aus einem Minimalwert und einem Maximalwert des Schwankungsbands berechnet werden. Das Schwankungsband kann beispielsweise +/- 1%, +/- 2% usw. betragen.

Es ist eine Weiterbildung, dass zur Durchführung der Kalibrierung ein Messwert Im des Pumpstroms bestimmt wird und überprüft wird, ob weitere in der vorgegebenen Zeitdauer ermittelte Messwerte Ip innerhalb des Schwankungsbands bleiben. Die obere Grenze und die untere Grenze des Schwankungsbands können bei einer Schwankungsbreite von +/m % durch 0,99 · Im bzw. 1,01 · Im bestimmt werden. Dabei müssen in einer Weiterbildung alle weiteren Messwerte Ip innerhalb des Schwankungsbands bleiben, also z.B. ob 0,99 · Im < Ip < 1,01 Im gilt. Ist dies der Fall, kann z.B. der Messwert Im als neuer Referenzwert verwendet werden, insbesondere als der einem Sauerstoffanteil von 20,95 % entsprechende Messwert. Ist dies nicht der Fall, kann der Kalibriervorgang abgebrochen oder wiederholt werden. Der Messwert Im kann z.B. ein erster Messwert sein.

Es ist zudem eine Ausgestaltung, dass der Kalibriervorgang abgebrochen wird, wenn er länger eine ein vorgegebener Schwellwert andauert, z.B. länger als zwei Minuten. So kann ein "Hängen" des Kalibriervorgangs bei wiederholt erfolglos durchgeführten Kalibrierversuchen verhindert werden.

Es ist auch noch eine Ausgestaltung, dass der Kalibriervorgang abgebrochen wird, wenn die Garraumtür geschlossen wird. Dies erhöht eine Zuverlässigkeit des Kalibriervorgangs weiter.

Es ist zudem noch eine Ausgestaltung, dass die Ergebnisse der Kalibriervorgänge gespeichert werden und auf eine Fehlfunktion des Sauerstoffsensors hin ausgewertet werden. Dies ermöglicht vorteilhafterweise eine frühzeitige Erkennung einer eventuellen Fehlfunktion des Sauerstoffsensors. Das Ergebnis des Kalibriervorgangs kann ein neuer Ergebniswert sein. Das Auswerten kann z.B. ein Vergleichen des neuen bzw. aktuellen Ergebniswerts mit mindestens einem vorher bestimmten (historischen) Ergebniswert umfassen. Beispielsweise kann eine Fehlfunktion festgestellt werden, wenn der aktuelle Ergebniswert um mehr als eine vorgegebene Differenz zu dem zuletzt gültigen Ergebniswert oder zu einem Mittelwert einer Gruppe abgespeicherter Ergebniswerte abweicht. Auch kann z.B. eine Fehlfunktion festgestellt werden, wenn ein aktueller Ergebniswerts von einem historischen Trend der Ergebniswerte abweicht, z.B. nicht wie bisher absinkt, sondern ansteigt usw.

Es ist noch eine Ausgestaltung, dass als Ergebnisse die Referenzwerte des Pumpstroms einer Lambdasonde verwendet werden.

Die Aufgabe wird auch gelöst durch ein Haushaltsgerät, aufweisend einen Behandlungsraum, einen Sauerstoffsensor und eine Datenverarbeitungseinrichtung zum Bestimmen eines Feuchtegehalts in dem Behandlungsraum beruhend auf Messwerten des Sauerstoffsensor, wobei das Haushaltsgerät zum Kalibrieren der Messwerte gemäß dem oben beschriebenen Verfahren eingerichtet ist. Das Haushaltsgerät kann analog zu dem Verfahren ausgebildet sein und weist die gleichen Vorteile auf.

Es ist eine Ausgestaltung, dass das Haushaltsgerät ein Dampfgargerät ist, das als den Behandlungsraum einen mit Dampf beaufschlagbaren Garraum aufweist.

Es ist eine Weiterbildung, dass die Datenverarbeitungseinrichtung dazu eingerichtet ist, das Verfahren durchzuführen. Die Datenverarbeitungsvorrichtung kann auch dazu eingerichtet sein, weitere Funktionen des Haushaltsgeräts zu steuern, z.B. Garprogramme oder andere Programme wie Reinigungsprogramme usw. Die Datenverarbeitungsvorrichtung kann eine Steuereinrichtung des Haushaltsgeräts sein.

Es ist noch eine Weiterbildung, dass die Datenverarbeitungseinrichtung einen Speicher ausweist oder mit einem Speicher gekoppelt ist, in dem Ergebnisse von Kalibriervorgängen (Historie der Ergebnisse) abspeicherbar sind. Der Speicher kann insbesondere ein EEPROM oder ein anderer nichtflüchtiger, insbesondere löschbarer, Speicher sein.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden schematischen Beschreibung eines Ausführungsbeispiels, das im Zusammenhang mit den Zeichnungen näher erläutert wird.

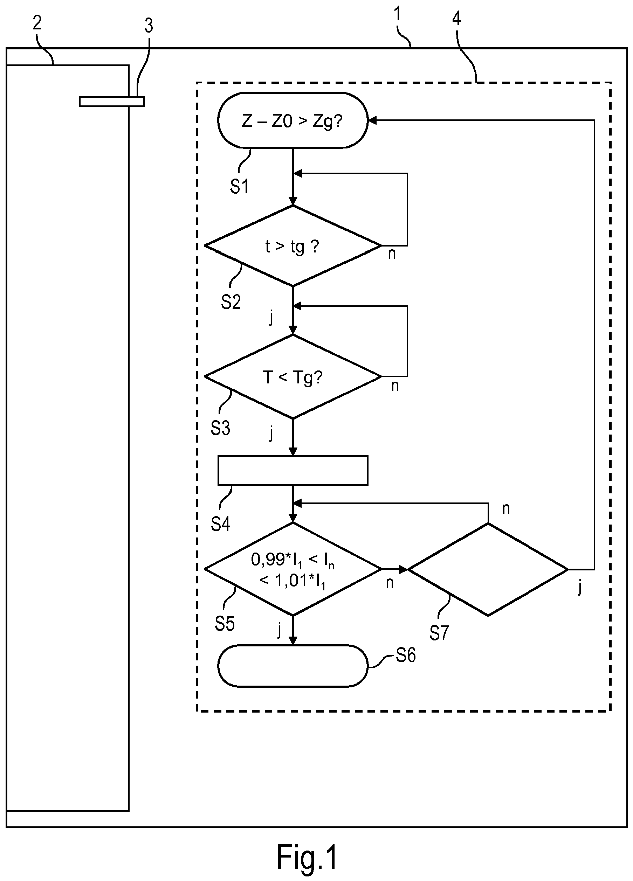

- Fig.1: zeigt dazu ein mögliches Ablaufdiagramm eines Verfahrens zum Kalibrieren von Messwerten eines Sauerstoffsensors eines Dampfgargeräts.

Das Haushaltsgerät in Form eines Dampfgargeräts 1 weist einen mit Dampf beaufschlagbaren Garraum 2, einen Sauerstoffsensor in Form einer Lambdasonde 3 und eine Datenverarbeitungseinrichtung 4 auf. Die Datenverarbeitungseinrichtung 4 ist dazu eingerichtet, einen Feuchtegehalt in dem Garraum 2 beruhend auf Messwerten der Lambdasonde 3 zu bestimmen. Die Datenverarbeitungseinrichtung 4 ist ferner zum Durchführen des Verfahrens eingerichtet. Die Datenverarbeitungseinrichtung 4 kann eine zentrale Steuereinrichtung zum Betreiben des Dampfgargeräts 1 sein.

Bei dem Verfahren überprüft die Datenverarbeitungseinrichtung 4 in einem ersten Schritt S1, ob ein Zustandsparameter in Form einer Zeitdauer Z - Z0 seit dem Zeitpunkt Z0 einer letzten Kalibrierung größer ist als ein Schwellwert in Form einer vorgegebenen Zeitdauer Zg. In anderen Worten wird in Schritt S1 überprüft, ob von der zum Zeitpunkt Z0 durchgeführten letzten Kalibrierung bis zum aktuellen Zeitpunkt Z bereits die Zeitdauer Zg vergangen ist. Zg kann z.B. 30 Stunden betragen. Ist dies nicht der Fall, wird Schritt S1 zu einem späteren Zeitpunkt wiederholt. Der Schritt S1 kann von der Datenverarbeitungseinrichtung 4 regelmäßig durchgeführt werden. Ist dies jedoch der Fall, wird ein Schritt S2 durchgeführt. Die Zeitpunkte Z und Z0 bzw. die Zeitdauer Zg können beispielsweise mittels eines Betriebsstundenzählers des Dampfgargeräts 1 erfasst werden.

In dem Schritt S2 wird überprüft, ob eine Türöffnungszeit t einer Garraumtür einen Schwellwert in Form einer vorgegebenen Zeitdauer tg überschritten hat, hier z.B. eine Zeitdauer tg von 60 Sekunden. Ist dies nicht der Fall ("n"), wird Schritt S2 wiederholt. Ist dies jedoch der Fall ("j"), wird ein Schritt S3 durchgeführt.

In dem Schritt S3 wird überprüft, ob eine Garraumtemperatur T einen Schwellwert in Form einer vorgegebenen Grenztemperatur Tg unterschritten hat, hier z.B. eine Grenztemperatur Tg von 50 °C. Ist dies nicht der Fall ("n"), wird Schritt S3 wiederholt. Alternativ könnte z.B. zu Schritt S2 zurückgekehrt werden. Ist dies jedoch der Fall ("j"), wird ein Schritt S4 durchgeführt.

Durch die drei Schritte S1 bis S3 ist die Wahrscheinlichkeit sehr hoch, dass die Lambdasonde 3 von frischer Umgebungsluft mit einem Sauerstoffanteil von ca. 20,95 % umgeben ist.

In dem Schritt S4 wird - da alle drei in den Schritten S1 bis S3 abgefragten Bedingungen vorliegen - der Kalibriervorgang automatisch durch die Datenverarbeitungseinrichtung 4 gestartet. Dazu wird in dem Schritt S4 die Lambdasonde 3 zunächst zur Aufnahme von Messungen vorbereitet, z.B. beheizt.

In einem Schritt S5 wird dann ein Pumpstrom I der Lambdasonde 3 gemessen. Und zwar wird zunächst ein erster Messwert I₁ aufgenommen. In folgenden Messungen, die z.B. in einem zeitlichen Abstand von fünf Sekunden erfolgen, werden die zugehörigen Messwerte Iₙ durch die Datenverarbeitungseinrichtung 4 daraufhin untersucht, ob sie alle innerhalb eines um den ersten Messwert I₁ herumgelegten Schwankungsbands von hier beispielhaft I₁ +/- 1 % liegen. Der Schritt S5 kann für eine vorgegebene Zeitdauer durchgeführt werden, z.B. für 30 Sekunden.

Fällt Schritt S5 erfolgreich aus ("j"), wird in einem folgenden Schritt S6 der erste Messwert I₁ als ein neuer Referenzwert des Pumpstroms I für einen Sauerstoffgehalt von 20,95 % angesehen. Der Kalibriervorgang wird damit beendet.

Fällt Schritt S5 erfolglos aus ("n"), kann folgend in einem Schritt S7 abgefragt ob der Kalibriervorgang abgebrochen werden soll. Ist dies der Fall ("j"), kann zur Überwachung von Schritt S1 übergegangen werden. Falls nicht ("n"), kann - ggf. mehrfach - zu Schritt S5 zurückgekehrt werden. Beispielsweise kann eine vorgegebene Zahl (z.B. von maximal vier Mal) von Kalibrierversuchen gemäß Schritt S5 durchgeführt werden und/oder versucht werden, Schritt S5 innerhalb einer vorgesehenen Zeitdauer (z.B. mit einem Schwellwert von zwei Minuten) erfolgreich zu beenden, bevor zu Schritt S7 übergegangen wird.

Die Abfrage in Schritt S7 kann auch darauf gerichtet sein, ob vor erfolgreicher Durchführung die Garraumtür wieder geschlossen worden ist.

Die Datenverarbeitungseinrichtung 4 kann auch dazu eingerichtet sein, die Referenzwerte zu speichern und somit eine Historie dieser Referenzwerte anzulegen. Die Datenverarbeitungseinrichtung 4 kann ferner dazu eingerichtet sein, die Referenzwerte- beispielsweise durch einen Vergleich mindestens eines zuvor bestimmten Referenzwerts mit dem aktuell gemessenen Referenzwert - dazu zu nutzen, auf eine Fehlfunktion des Sauerstoffsensors hin auszuwerten.

Selbstverständlich ist die vorliegende Erfindung nicht auf das gezeigte Ausführungsbeispiel beschränkt.

Allgemein kann unter "ein", "eine" usw. eine Einzahl oder eine Mehrzahl verstanden werden, insbesondere im Sinne von "mindestens ein" oder "ein oder mehrere" usw., solange dies nicht explizit ausgeschlossen ist, z.B. durch den Ausdruck "genau ein" usw.

Auch kann eine Zahlenangabe genau die angegebene Zahl als auch einen üblichen Toleranzbereich umfassen, solange dies nicht explizit ausgeschlossen ist.

### Bezugszeichenliste

- 1: Dampfgargerät
- 2: Garraum
- 3: Lambdasonde
- 4: Datenverarbeitungseinrichtung
- S1-S5: Verfahrensschritte
- t: Türöffnungszeit
- tg: Vorgegebene Zeitdauer
- T: Garraumtemperatur
- Tg: Grenztemperatur
- Z: Aktueller Zeitpunkt
- Z0: Zeitpunkt einer letzten Kalibrierung
- Zg: Vorgegebene Zeitdauer
- I₁: Erster Messwert des Pumpstroms
- Iₙ: n-ter Messwert des Pumpstroms

## Patentansprüche

1. Verfahren (S1-S7) zum Starten eines Kalibriervorgangs (S4-S7) eines Sauerstoffsensors (3) eines Haushaltsgeräts (1) mit einem Behandlungsraum (2) und einer den Behandlungsraum (2) verschließenden Tür, bei dem der Kalibriervorgang (S4-S7) dann automatisch gestartet wird, wenn eine Türöffnungszeit (t) einen zugehörigen Schwellwert (tg) erreicht oder überschritten hat.

2. Verfahren (S1-S7) nach Anspruch 1, bei dem der Kalibriervorgang (S4-S7) dann gestartet wird, wenn zusätzlich mindestens ein weiterer Zustandsparameter (Z, t, T) des Haushaltsgeräts (1) einen jeweils vorgegebenen Schwellwert (Zg, tg, Tg) zumindest erreicht hat (S1-S3).

3. Verfahren (S1-S7) nach einem der vorhergehenden Ansprüche, bei dem das Haushaltsgerät (1) ein Dampfgargerät ist.

4. Verfahren (S1-S7) nach einem der vorhergehenden Ansprüche, bei dem als der Sauerstoffsensor (3) eine Lambdasonde verwendet wird.

5. Verfahren (S1-S7) nach einem der vorhergehenden Ansprüche, bei dem der mindestens eine weitere Zustandsparameter (Z, t, T) eine Zeitdauer (Z-ZO) seit der letzten Kalibrierung umfasst und der Kalibriervorgang (S4-S7) dann automatisch gestartet wird, wenn zusätzlich die Zeitdauer (Z-ZO) einen zugehörigen Schwellwert (Zg) erreicht oder überschritten hat.

6. Verfahren (S1-S7) nach einem der vorhergehenden Ansprüche, bei dem das Haushaltsgerät (1) ein Gargerät ist, der Behandlungsraum (2) ein Garraum ist und der mindestens eine weitere Zustandsparameter (Z, t, T) eine Garraumtemperatur (T) umfasst und der Kalibriervorgang (S4-S7) dann automatisch gestartet wird, wenn zusätzlich die Garraumtemperatur (T) einen zugehörigen Schwellwert (Tg) erreicht oder unterschritten hat.

7. Verfahren (S1-S7) nach einem der vorhergehenden Ansprüche, bei dem das Haushaltsgerät (1) einen Nutzer auffordert, das Verfahren (S1-S7) durchzuführen und dazu ein Tür für eine angezeigte Zeitdauer bei kühlem Garraum zu öffnen. wenn es dem Haushaltsgerät (1) nicht möglich war, das Verfahren (S1-S7) innerhalb einer vorgegebenen Zeitdauer erfolgreich durchzuführen.

8. Verfahren (S1-S7) nach einem der vorhergehenden Ansprüche, bei dem während des Kalibriervorgangs (S4-S7) ein Pumpstrom (I₁, Iₙ) einer als Sauerstoffsensor (3) dienenden Lambdasonde gemessen wird und dann, wenn der Pumpstrom (I₁, Iₙ) für eine vorgegebene Zeitdauer innerhalb eines vorgegebenen Schwankungsbands verbleibt, ein Wert des Pumpstroms (I₁) aus diesem Schwankungsband als ein Referenzwert des Pumpstroms ausgewählt wird.

9. Verfahren (S1-S7) nach Anspruch 8, bei dem ein mittlerer Wert (I₁) des Schwankungsbands als der Referenzwert des Pumpstroms ausgewählt wird.

10. Verfahren (S1-S7) nach einem der vorhergehenden Ansprüche, bei dem der Kalibriervorgang (S4-S7) abgebrochen wird (S7), wenn er länger als ein vorgegebener Schwellwert andauert.

11. Verfahren (S1-S7) nach einem der vorhergehenden Ansprüche 6 bis 10, bei dem der Kalibriervorgang (S4-S7) abgebrochen wird (S7), wenn eine Garraumtür geschlossen wird.

12. Verfahren (S1-S7) nach einem der vorhergehenden Ansprüche, bei dem Ergebnisse (I₁) der Kalibriervorgänge (S4-S7) gespeichert werden und auf eine Fehlfunktion des Sauerstoffsensors (3) hin ausgewertet werden.

13. Verfahren (S1-S7) nach den Ansprüchen 9 und 12, bei dem als Ergebnisse (I₁) die Referenzwerte des Pumpstroms verwendet werden.

14. Haushaltsgerät (1), aufweisend einen Behandlungsraum (2), einen Sauerstoffsensor (3) und eine Datenverarbeitungseinrichtung (4) zum Bestimmen eines Feuchtegehalts in dem Behandlungsraum (2) beruhend auf Messwerten (I₁, Iₙ) des Sauerstoffsensors (3), **dadurch gekennzeichnet, dass** das Haushaltsgerät (1) zum Kalibrieren der Messwerte (I₁, Iₙ) gemäß dem Verfahren (S1-S7) nach einem der vorhergehenden Ansprüche eingerichtet ist.

15. Haushaltsgerät (1) nach Anspruch 14, **dadurch gekennzeichnet, dass** das Haushaltsgerät (1) ein Dampfgargerät ist, das als den Behandlungsraum (2) einen mit Dampf beaufschlagbaren Garraum (2) aufweist.

## Claims

1. Method (S1-S7) for starting a calibration process (S4-S7) of an oxygen sensor (3) of a household appliance (1) with a treatment chamber (2) and a door closing off the treatment chamber (2), in which the calibration process (S4-S7) is automatically started when a door opening time (t) has reached or exceeded an associated threshold value (tg).

2. Method (S1-S7) according to claim 1, in which the calibration process (S4-S7) is started when additionally at least one further status parameter (Z, t, T) of the household appliance (1) has at least reached (S1-S3) a respective predetermined threshold value (Zg, tg, Tg).

3. Method (S1-S7) according to one of the preceding claims, in which the household appliance (1) is a steam cooking appliance.

4. Method (S1-S7) according to one of the preceding claims, in which a lambda probe is used as the oxygen sensor (3).

5. Method (S1-S7) according to one of the preceding claims, in which the at least one further status parameter (Z, t, T) comprises a time period (Z-ZO) since the last calibration and the calibration process (S4-S7) is automatically started when additionally the time period (Z-ZO) has reached or exceeded an associated threshold value (Zg).

6. Method (S1-S7) according to one of the preceding claims, in which the household appliance (1) is a cooking appliance, the treatment chamber (2) is a cooking chamber and the at least one further status parameter (Z, t, T) comprises a cooking chamber temperature (T) and the calibration process (S4-S7) is automatically started when additionally the cooking chamber temperature (T) has reached or fallen below an associated threshold value (Tg).

7. Method (S1-S7) according to one of the preceding claims, in which the household appliance (1) prompts a user to execute the method (S1-S7) and to this end to open a door for a displayed time period when the cooking chamber is cool, if it had not been possible for the household appliance (1) to execute the method (S1-S7) successfully within a predetermined time period.

8. Method (S1-S7) according to one of the preceding claims, in which during the calibration process (S4-S7) a pump flow (I₁, Iₙ) of a lambda probe serving as an oxygen sensor (3) is measured and when the pump flow (I₁, Iₙ) remains for a predetermined time period within a predetermined fluctuation margin, a value of the pump flow (I₁) is selected from this fluctuation margin as a reference value of the pump flow.

9. Method (S1-S7) according to claim 8, in which an average value (I₁) of the fluctuation margin is selected as the reference value of the pump flow.

10. Method (S1-S7) according to one of the preceding claims, in which the calibration process (S4-S7) is discontinued (S7) if it lasts longer than a predetermined threshold value.

11. Method (S1-S7) according to one of the preceding claims 6 to 10, in which the calibration process (S4-S7) is discontinued (S7) if a cooking chamber door is closed.

12. Method (S1-S7) according to one of the preceding claims, in which results (I₁) of the calibration processes (S4-S7) are stored and evaluated for a malfunction of the oxygen sensor (3).

13. Method (S1-S7) according to claim 9 and 12, in which the reference values of the pump flow are used as results (I₁).

14. Household appliance (1) comprising a treatment chamber (2), an oxygen sensor (3) and a data processing device (4) for determining a moisture content in the treatment chamber (2) based on measured values (I₁, Iₙ) of the oxygen sensor (3), **characterised in that** the household appliance (1) is designed for calibrating the measured values (I₁, Iₙ) according to the method (S1-S7) according to one of the preceding claims.

15. Household appliance (1) according to claim 14, **characterised in that** the household appliance (1) is a steam cooking appliance which has a cooking chamber (2) as the treatment chamber (2), to which steam is able to be applied.

## Revendications

1. Procédé (S1-S7) de démarrage d'une opération d'étalonnage (S4-S7) d'un capteur d'oxygène (3) d'un appareil électroménager (1) comprenant une chambre de traitement (2) et une porte fermant la chambre de traitement (2), dans lequel l'opération d'étalonnage (S4-S7) est démarrée automatiquement lorsqu'une durée d'ouverture de porte (t) a atteint ou dépassé une valeur seuil (tg) correspondante.

2. Procédé (S1-S7) selon la revendication 1, dans lequel l'opération d'étalonnage (S4-S7) est démarrée lorsqu'en outre au moins un paramètre d'état supplémentaire (Z, t, T) de l'appareil électroménager (1) a au moins atteint une valeur seuil (Zg, tg, Tg) respectivement prédéterminée.

3. Procédé (S1-S7) selon l'une des revendications précédentes, dans lequel l'appareil électroménager (1) est un appareil de cuisson à la vapeur.

4. Procédé (S1-S7) selon l'une des revendications précédentes, dans lequel, en tant que capteur d'oxygène (3), une sonde lambda est utilisée.

5. Procédé (S1-S7) selon l'une des revendications précédentes, dans lequel l'au moins un paramètre d'état supplémentaire (Z, t, T) comprend un intervalle de temps (Z-ZO) depuis le dernier étalonnage et l'opération d'étalonnage (S4-S7) est automatiquement démarrée lorsqu'en outre l'intervalle de temps (Z-ZO) a atteint ou dépassé une valeur seuil (Zg) correspondante.

6. Procédé (S1-S7) selon l'une des revendications précédentes, dans lequel l'appareil électroménager (1) est un appareil de cuisson, la chambre de traitement (2) est une chambre de cuisson et l'au moins un paramètre d'état supplémentaire (Z, t, T) comprend une température de chambre de cuisson (T) et l'opération d'étalonnage (S4-S7) est automatiquement démarrée lorsqu'en outre la température de chambre de cuisson (T) a atteint ou dépassé une valeur seuil (Tg) correspondante.

7. Procédé (S1-S7) selon l'une des revendications précédentes, dans lequel l'appareil électroménager (1) invite un utilisateur à exécuter le procédé (S1-S7) et à ouvrir à cet effet une porte pendant une durée indiquée lorsque la chambre de cuisson est froide, lorsqu'il n'a pas été possible à l'appareil électroménager (1) d'exécuter le procédé (S1-S7) avec succès dans un intervalle de temps prédéterminé.

8. Procédé (S1-S7) selon l'une des revendications précédentes, dans lequel, pendant l'opération d'étalonnage (S4-S7), un courant de pompage (I₁, Iₙ) d'une sonde lambda servant de capteur d'oxygène (3) est mesuré, et lorsque le courant de pompage (I₁, Iₙ) demeure pendant un intervalle de temps prédéterminé dans une plage de variation prédéterminée, une valeur du courant de pompage (I₁) est sélectionnée à partir de cette plage de variation à titre de valeur de référence du courant de pompage.

9. Procédé (S1-S7) selon la revendication 8, dans lequel une valeur centrale (I₁) de la plage de variation est sélectionnée en tant que valeur de référence du courant de pompage.

10. Procédé (S1-S7) selon l'une des revendications précédentes, dans lequel l'opération d'étalonnage (S4-S7) est interrompue (S7) lorsqu'elle dure plus longtemps qu'une valeur seuil prédéterminée.

11. Procédé (S1-S7) selon l'une des revendications 6 à 10, dans lequel l'opération d'étalonnage (S4-S7) est interrompue (S7) lorsqu'une porte de chambre de cuisson est fermée.

12. Procédé (S1-S7) selon l'une des revendications précédentes, dans lequel des résultats (I₁) des opérations d'étalonnage (S4-S7) sont enregistrés et sont analysés en ce qui concerne un défaut de fonctionnement du capteur d'oxygène (3).

13. Procédé (S1-S7) selon l'une des revendications 9 et 12, dans lequel les valeurs de référence du courant de pompage sont utilisées à titre de résultats (I₁).

14. Appareil électroménager (1), comprenant une chambre de traitement (2), un capteur d'oxygène (3) et un dispositif de traitement de données (4) pour déterminer une teneur en humidité dans la chambre de traitement (2) sur la base de valeurs de mesure (I₁, Iₙ) du capteur d'oxygène (3)
**caractérisé en ce que** l'appareil électroménager (1) est configuré pour étalonner les valeurs de mesure (I₁, Iₙ) selon le procédé (S1-S7) selon l'une des revendications précédentes.

15. Appareil électroménager (1) selon la revendication 14, **caractérisé en ce que** l'appareil électroménager (1) est un appareil de cuisson à la vapeur, qui comprend, à titre de chambre de traitement (2), une chambre de cuisson (2) susceptible d'être soumise à de la vapeur.
